(19)

Europäisches Patentamt

European Patent Office

Office européen des brevets

(11) **EP 4 681 805 A1**

(12) **EUROPEAN PATENT APPLICATION**
published in accordance with Art. 153(4) EPC

(43) Date of publication:
**21.01.2026 Bulletin 2026/04**

(21) Application number: **25784933.1**

(22) Date of filing: **07.05.2025**

(51) International Patent Classification (IPC):
**B01F 21/00** *(2022.01)*

(52) Cooperative Patent Classification (CPC):
**A61K 9/51; B01J 19/00; B01L 9/00**

(86) International application number:
**PCT/KR2025/006132**

(87) International publication number:
**WO 2025/234761 (13.11.2025 Gazette 2025/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **07.05.2024 KR 20240060013**

(71) Applicant: **Mepsgen Co., Ltd.
Seoul 05836 (KR)**

(72) Inventors:
• **KIM, Yong Tae**
  **Seoul 05855 (KR)**
• **JEON, Mi Yeon**
  **Seoul 05667 (KR)**
• **LEE, Woo Seung**
  **Seoul 05667 (KR)**

(74) Representative: **Hauck Patent- und
Rechtsanwälte PartmbB
Am Sandtorkai 68
20457 Hamburg (DE)**

(54) **AUTOMATED APPARATUS FOR PRODUCING NANOPARTICLES**

(57)    The present invention relates to an automated apparatus for manufacturing nanoparticles and a method for manufacturing nanoparticles using the same. Specifically, the present invention relates to an automated apparatus for manufacturing nanoparticles, including: a microfluidic device; a mounting unit for securing the microfluidic device to the automated apparatus; an inlet unit for supplying a fluid containing raw materials; a pneumatic control unit for providing pressure for fluid movement; and a collection unit for collecting manufactured nanoparticles under controlled conditions.

【FIG.2】

EP 4 681 805 A1

## Description

[Technical Field]

The present invention relates to an automated apparatus for manufacturing nanoparticles, including lipid nanoparticles (LNPs). Specifically, the present invention relates to an automated apparatus for manufacturing nanoparticles, which includes a microfluidic device, a mounting unit for securing the microfluidic device to the automated apparatus, an inlet unit for supplying a fluid containing raw materials, a pneumatic control unit for providing pressure for fluid movement, and a collection unit for collecting the manufactured nanoparticles under specific conditions.

[Background Art]

Many nanomedicines are being developed for targeted delivery of therapeutics and imaging agents for the treatment and diagnosis of major diseases including cancer, cardiovascular disease, diabetes, and Alzheimer's disease. Effective drug delivery systems can improve the absorption of poorly soluble and unstable drugs while increasing the therapeutic efficacy of drugs and reducing the toxic effects of drugs. Furthermore, this has led to the discovery and development of more effective drugs for improving patient prognosis and quality of life.

In general, nanoparticles developed as pharmaceuticals are based on phospholipid-based lipid nanoparticles. In the early stages of technology development, emulsion or extrusion methods were commonly used. However, these methods resulted in low yields and produced non-uniform particle sizes, thereby limiting their industrial value. More recently, microfluidic manufacturing methods have become the preferred alternative to conventional lipid nanoparticle synthesis. Such methods enable more efficient nanoparticle production by implementing micrometer-scale microstructures and maximizing the interface between hydrophobic fluids containing phospholipids and hydrophilic fluids containing pharmacological ingredients.

Currently, the most commonly used microstructure mixers are T (or Y) mixers or ring mixers. However, the method for manufacturing nanoparticles using microstructures requires a high level of automation for mass production. To this end, equipment for automated nanoparticle production using microfluidic devices has recently been developed. However, the most important aspect of automated nanoparticle manufacturing using microfluidic devices is the ability to mass-produce small and uniform nanoparticles.

Therefore, the inventors of the present invention have continuously conducted research to achieve the above object and, recognizing the need for a precisely calculated constant flow rate, have introduced a pressure control method into an automated apparatus for manufacturing nanoparticles, thereby completing the present invention.

[Prior Art Document]

(Patent Document 1) Korean Patent No. 10-2492420

(Non-Patent Document 1) Valencia, P. et al., Single-Step Assembly of Homogenous Lipid-Polymeric and Lipid-Quantum Dot Nanoparticles Enabled by Microfluidic Rapid Mixing, ACS Nano, 4(3), 1671-1679 (2010)

(Non-patent Document 2) Rhee, M. et al. Drop Mixing in a Microchannel for Lab-on-a-Chip Platforms. Langmuir, 24(2), 590-601 (2008)

(Non-patent Document 3) Rohit, K et al., Microfluidic Platform for Controlled Synthesis of Polymeric Nanoparticles. Nano Letters, 8(9), 2906-12, (2008).

[Summary of Invention]

[Problems to be Solved by Invention]

It is an object of the present invention to mass-produce uniform nanoparticles using a microfluidic device designed to maximize the mixing of different fluids.

Specifically, it is another object of the present invention to provide an automated apparatus for manufacturing nanoparticles that precisely controls the fluid flow rate using a raw material injection device controlled by a high-precision pneumatic control unit, and automatically sorts each nanoparticle synthesized under various conditions using a rotary sample collection unit.

[Means for Solving Problems]

The present invention provides an apparatus for manufacturing nanoparticles including: a microfluidic device; an inlet unit; a collection unit; and a pneumatic control unit.

The apparatus for manufacturing nanoparticles of the present invention may further include a microfluidic device mounting unit.

The apparatus for manufacturing nanoparticles of the present invention may further include a cabinet.

The apparatus for manufacturing nanoparticles of the present invention may further include a power supply.

The microfluidic device mounting unit of the present invention may include at least one selected from the group consisting of a mounting unit cover, a microfluidic device securing holder, and an injection valve. Here, the injection valve may control the injection of a fluid transferred from the inlet unit.

The inlet unit of the present invention may include at least one selected from the group consisting of a tube mounting unit, a raw material injection tube, a sealing electric cylinder, a sealing gasket, a raw material tube mounting unit opening/closing electric cylinder, and a pneumatic fitting.

Here, the raw material injection tube may be sealed from outside air by the sealing electric cylinder and the sealing gasket. In addition, the pressure of a fluid controlled by the pneumatic control unit may be transmitted to the microfluidic device through the pneumatic fitting and the raw material injection tube. Further, the raw material tube mounting unit may be moved forward and backward by the raw material tube mounting unit opening/closing electric cylinder to open and close a door.

The collection unit of the present invention may include at least one selected from the group consisting of a tube mounting unit, a rotation motor, a collection valve, and a collection tube mounting unit opening/closing electric cylinder.

Here, the collection tube mounting unit may be moved forward and backward by the collection tube mounting unit opening/closing electric cylinder to open and close a door. In addition, the collection tube mounting unit is rotated by the rotation motor, and nanoparticles synthesized in the microfluidic device may be stored in a separate collection tube.

The pneumatic control unit of the present invention may include at least one selected from the group consisting of a primary pneumatic control unit and a secondary pneumatic control unit.

In the apparatus for manufacturing nanoparticles, the microfluidic device may be secured to a microfluidic device securing holder.

In the apparatus for manufacturing nanoparticles of the present invention, the inlet unit and the collection unit may have an openable door structure.

In the apparatus for manufacturing nanoparticles of the present invention, a gas at a constant pressure may be supplied to the inlet unit through the primary pneumatic control unit and the secondary pneumatic control unit. Here, the gas may be supplied from outside of the apparatus.

In the apparatus for manufacturing nanoparticles of the present invention the microfluidic device may include one or more microchannels. The height-to-width ratio of the microchannel may be 1:1 to 1:5, and preferably, 1:1 to 1:3.

The Reynolds number in the microchannel may be 10 to 500, and preferably, 20 to 300. Specifically, when the height and width of the microchannel are 1:1 (X1), the Reynolds number may be 20 or more, preferably 20 to 300. Further, when the height and width of the microchannel are 1:3 (X3), the Reynolds number may be 150 or more, preferably 150 to 300.

The nanoparticles manufactured by the apparatus for manufacturing nanoparticles of the present invention have a size of 10 to 100 nm, preferably 30 to 90 nm, and more preferably 50 to 80 nm.

The polydispersity index of the nanoparticles manufactured by the apparatus for manufacturing nanoparticles of the present invention may be 0.05 to 0.15, preferably 0.10 to 0.15.

The encapsulation efficiency of nanoparticles manufactured by the apparatus for manufacturing nanoparticles of the present invention may be 70% or more, preferably 80% or more, and more preferably 90% or more.

The present invention provides a method for manufacturing nanoparticles using the apparatus for manufacturing nanoparticles of the present invention, the method including: introducing a fluid into the microfluidic device from the inlet unit at a constant flow rate; and collecting products produced in the microfluidic device in the collection unit.

Here, as the fluid is introduced into the microfluidic device from the inlet unit, the flow rate of the fluid may be controlled by the pneumatic control unit. In addition, the products may be separated and collected in the collection unit.

The apparatus for manufacturing nanoparticles may be placed in a cabinet.

[Advantageous Effects]

The automated apparatus for manufacturing nanoparticles according to the present invention can supply a fluid containing raw materials at a precisely controlled flow rate to a microfluidic device designed to generate micro-vortices, thereby enabling the uniform synthesis of nanoparticles of a desired size.

In addition, the apparatus can automatically replace collection tubes using a rotating rotor to separate nanoparticles under various conditions, thereby allowing identification of the optimal combination of precursors for the development of desired nanoparticles.

Accordingly, the apparatus of the present invention enables efficient development and large-scale production of nanoparticles for various purposes, while minimizing development costs and time and facilitating mass production.

[Brief Description of Drawings]

FIG. 1 is a perspective view of an apparatus for manufacturing nanoparticles ("nanoparticle manufacturing apparatus").

FIG. 2 is a perspective view of the nanoparticle manufacturing apparatus with doors of an inlet unit and a collection unit open.

FIG. 3 is a perspective view showing internal components of the nanoparticle manufacturing apparatus with the cabinet removed.

FIG. 4 is a perspective view of a microfluidic device mounting unit with a mounting unit cover open.

FIG. 5 is a bottom perspective view of the microfluidic device mounting unit.

FIG. 6 is a front view of the inlet unit.

FIG. 7 is a perspective view of the inlet unit.

FIG. 8 is a set of front and side views of the collection unit.

FIG. 9 is a plan view of the collection unit.

FIG. 10 is a perspective view of the collection unit.

FIG. 11 is a graph comparing the sizes of LNPs at lipid mixture concentrations of 0.66, 6.6, 13, and 20 mg/mL.

FIG. 12 is a graph comparing the polydispersity index (PDI) of LNPs at lipid mixture concentrations of 0.66, 6.6, 13, and 20 mg/mL.

FIG. 13 is a graph comparing the encapsulation efficiency (EE) of LNPs at lipid mixture concentrations of 0.66, 6.6, 13, and 20 mg/mL.

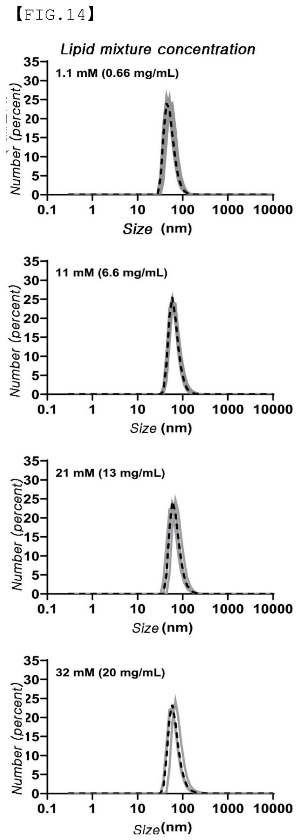

FIG. 14 is a set of graphs showing the DLS results for LNP size, expressed as number percent, at lipid mixture concentrations of 0.66, 6.6, 13, and 20 mg/mL.

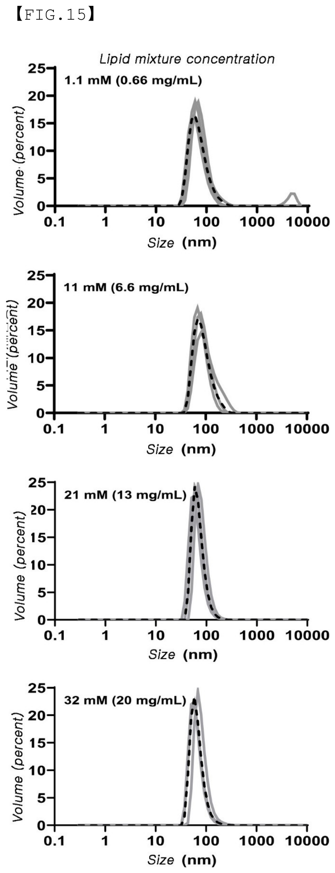

FIG. 15 is a set of graphs showing the DLS results for LNP size, expressed as volume percent, at lipid mixture concentrations of 0.66, 6.6, 13, and 20 mg/mL.

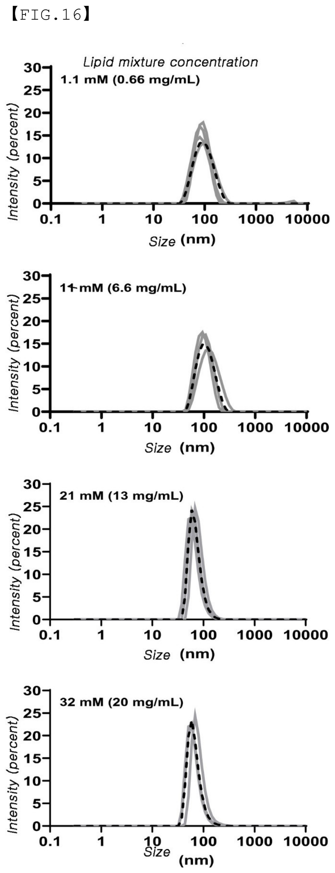

FIG. 16 is a set of graphs showing the DLS results for LNP size, expressed as intensity percent, at lipid mixture concentrations of 0.66, 6.6, 13, and 20 mg/mL.

[Mode for Carrying out Invention]

Hereinafter, embodiments and examples of the present invention will be described in detail with reference to the accompanying drawings, so that those skilled in the art can readily practice the present invention. However, it should be understood that the present invention may be embodied in various forms and is not limited to the specific embodiments and examples described herein.

Throughout this specification, when a part is referred to as "including" a component, it should be understood that, unless explicitly stated otherwise, the term does not exclude the presence of other components but may further include additional components.

The present invention relates to a nanoparticle manufacturing apparatus including a microfluidic device, an inlet unit, a collection unit, and a pneumatic control unit.

The present invention relates to a method for manufacturing nanoparticles using the nanoparticle manufacturing apparatus, which includes the steps of introducing a fluid containing raw materials from the inlet unit into the microfluidic device at a constant flow rate via pneumatic pressure, and classifying and collecting products in the collection unit.

As used herein, the term "microfluidic device" refers to a device including a microchannel configured to allow a fluid to flow on a substrate made of various materials, including plastic, glass, metal, or silicon, and which may include an organic polymer.

As used herein, the term "microchannel" refers to a channel having dimensions on the order of millimeters, micrometers, or nanometers, through which a fluid can flow, and is also referred to as a "mixing channel" in the present invention.

The microfluidic device used in the present invention may be the device described in Korean Patent No. 10-2631907.

The term "resistance" as used herein refers to the pressure per unit volume experienced by a fluid, proportional to time, having dimensions of Pascal-second per cubic meter ($Pa \cdot s/m^3$), when a fluid containing raw materials for nanoparticle production is injected into the microchannel of the microfluidic device at a constant flow rate.

The term "pressure" or "pneumatic pressure" as used herein refers to the pressure controlled during nanoparticle production, expressed in units of Pascal (Pa), kilopascal (kPa), millibar (mbar), or bar, and denotes the driving force that enables fluid injection and nanoparticle production through precise control.

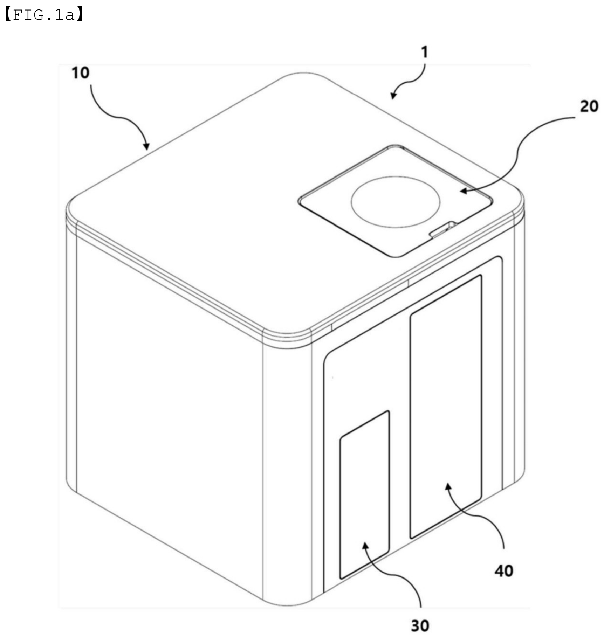

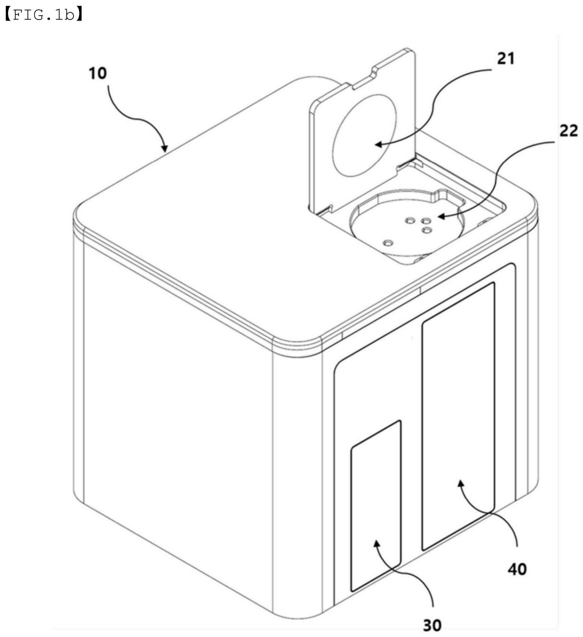

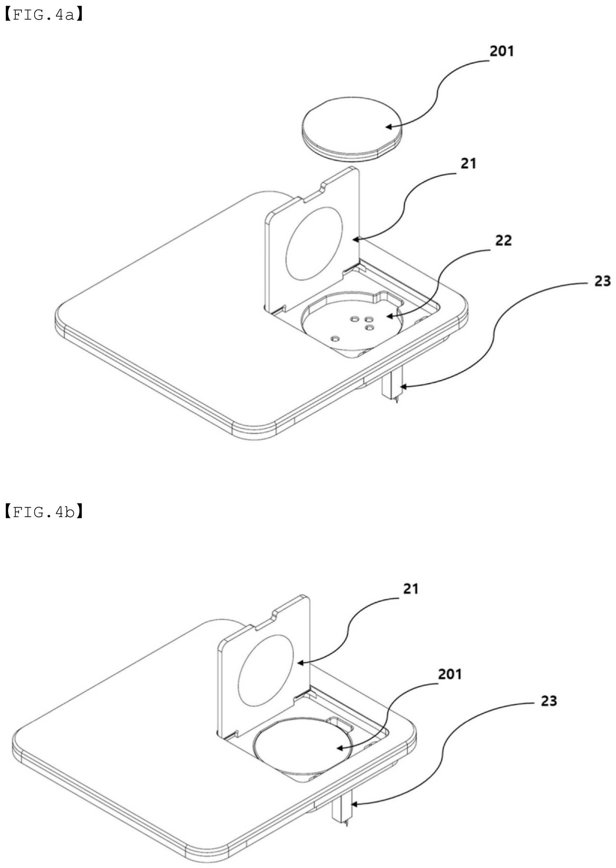

Hereinafter, the nanoparticle manufacturing apparatus of the present invention will be described in detail with reference to the accompanying drawings. The present invention may include the nanoparticle manufacturing apparatus embodied in the forms shown in FIGS. 1 to 10, but is not limited thereto, and may encompass various modifications implemented by those skilled in the art to which the present invention pertains.

A nanoparticle manufacturing apparatus 1 of the present invention includes a microfluidic device mounting unit 20, an inlet unit 30, a collection unit 40, and a pneumatic control unit 50.

Referring to FIG. 1, in the nanoparticle manufacturing apparatus 1 of the present invention, a microfluidic device securing holder 22 may be secured to a microfluidic device 201.

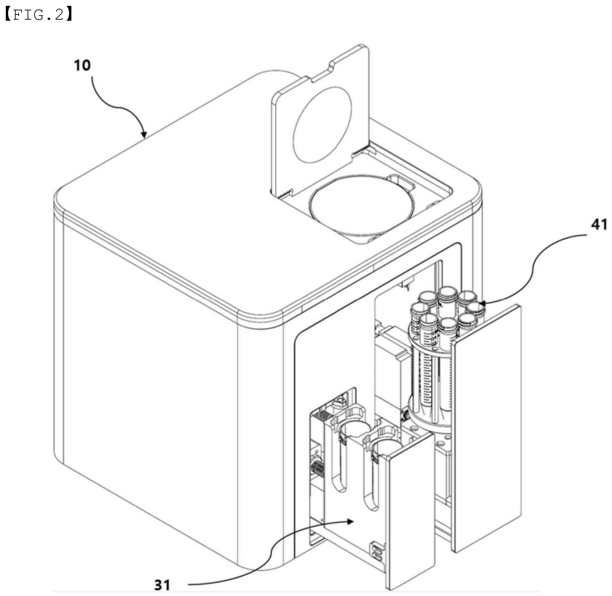

Referring to FIG. 2, the inlet unit 30 and the collection unit 40 have an open/close door structure, and each unit may include a raw material tube mounting unit 31 capable of securing tubes, and a rotary collection tube mounting unit 41.

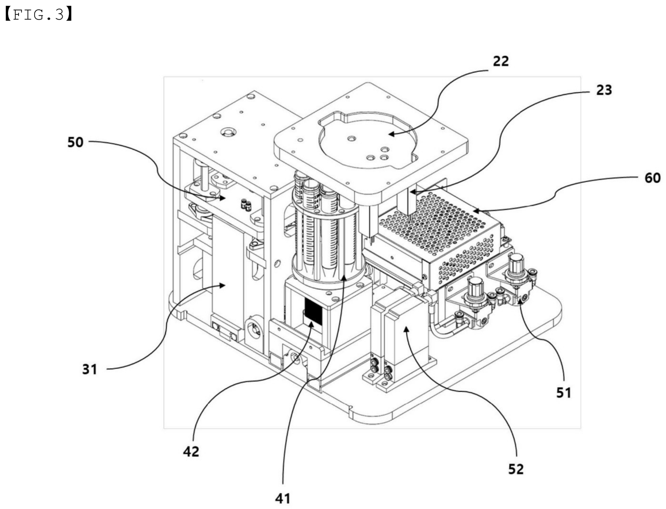

Referring to FIG. 3, the nanoparticle manufacturing apparatus 1 may include the pneumatic control unit 50, and may deliver a gas at a constant pressure to the inlet unit 30 through a primary pneumatic control unit 51 and a secondary pneumatic control unit 52. At this time, the gas supplied to the pneumatic control unit 50 may be supplied from outside the apparatus.

The gas supply source supplied to the nanoparticle manufacturing apparatus 1 may include a gas storage tank.

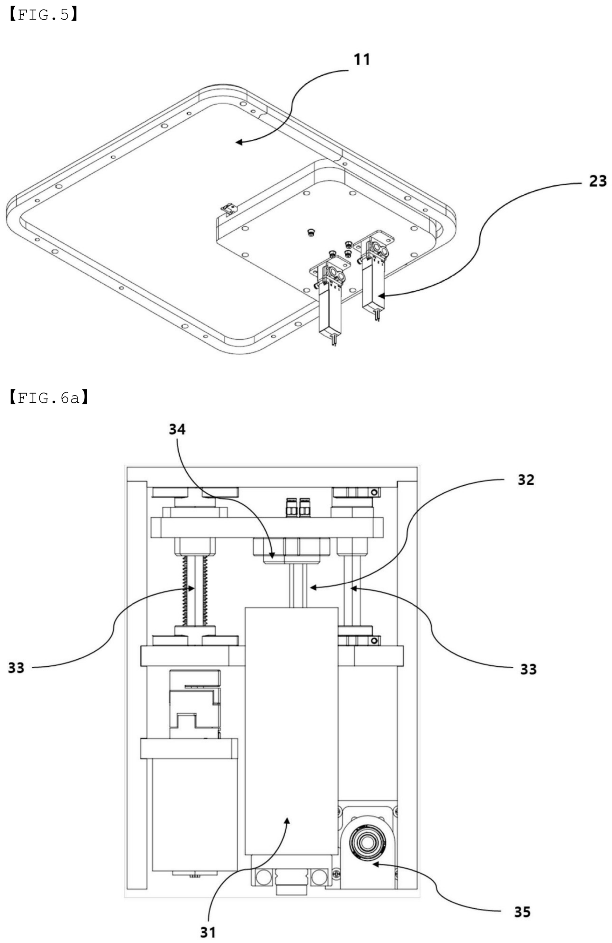

Referring to FIG. 5, the microfluidic device mounting unit 20 may include an injection valve 23 configured to control the injection of a fluid containing raw materials delivered from the inlet unit 30.

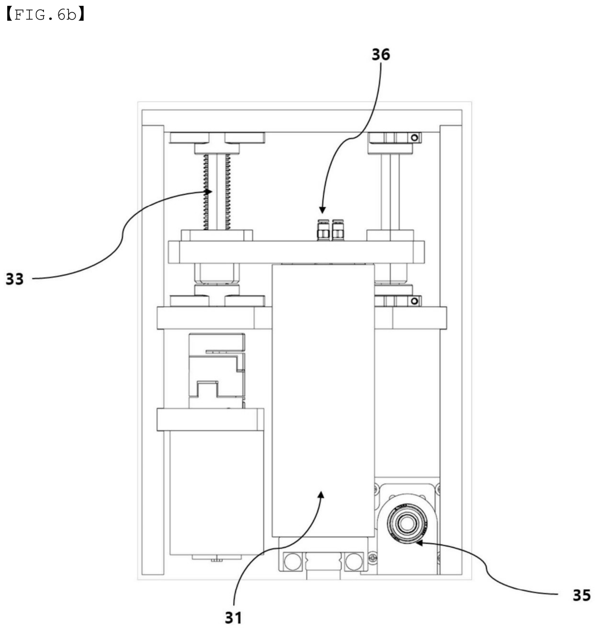

Referring to FIG. 6, the raw material tube secured to the raw material tube mounting unit 31 may be sealed from outside air by a sealing electric cylinder 33 and a sealing gasket 34. Through a pneumatic fitting 36 connected to the secondary pneumatic control unit 52 and a raw material injection tube 32, a constant pressure may be applied to the fluid containing raw materials inside the tube and delivered to the microfluidic device 201. In the present invention, the raw material injection tube may include bilateral injection tubes and a central injection tube.

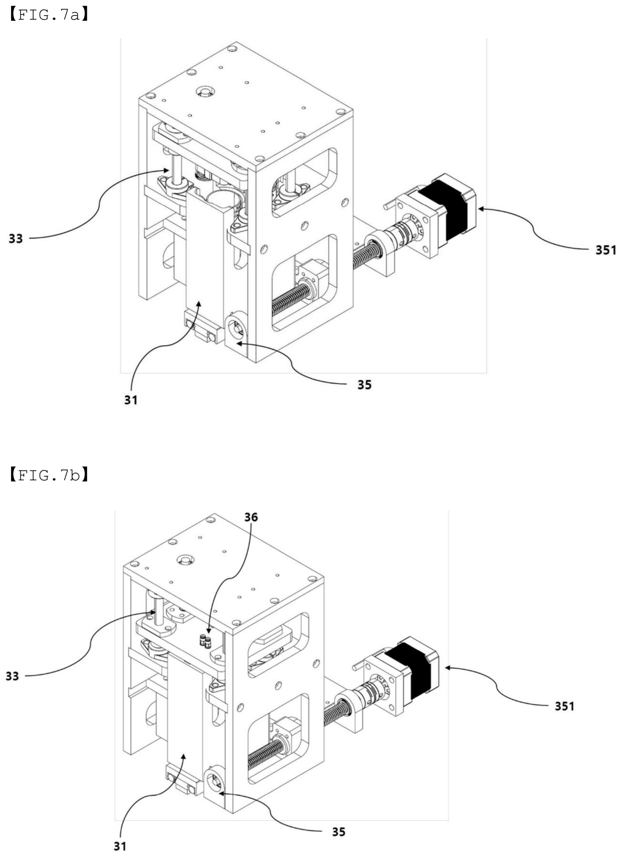

Referring to FIG. 7, the raw material tube mounting unit 31 may be moved forward and backward by a raw material tube mounting unit opening/closing electric cylinder 35 to open and close the door.

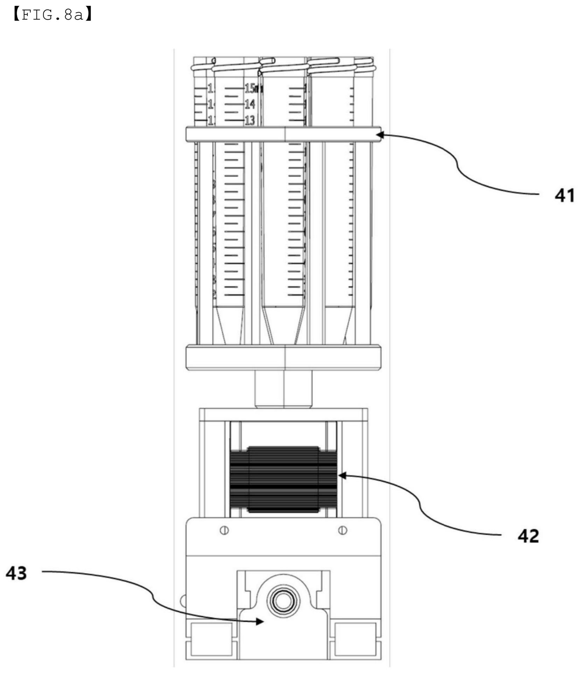

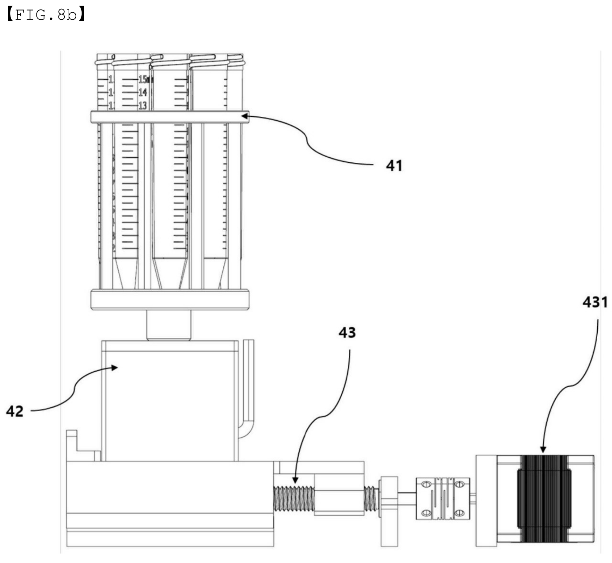

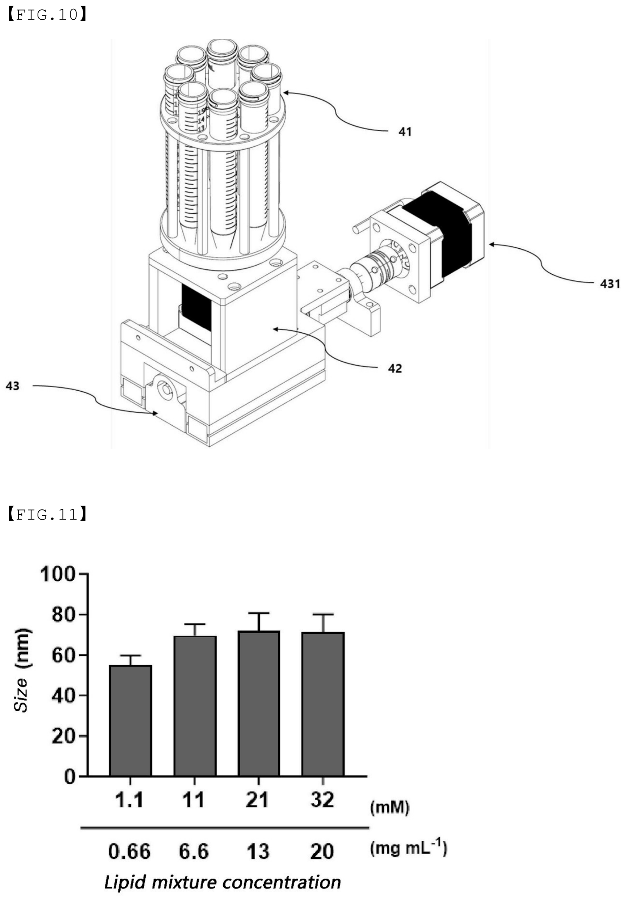

Referring to FIGS. 8 and 10, the rotary collection tube mounting unit 41 may be rotated by a rotation motor 42, thereby allowing nanoparticles synthesized under different conditions to be stored in different collection tubes.

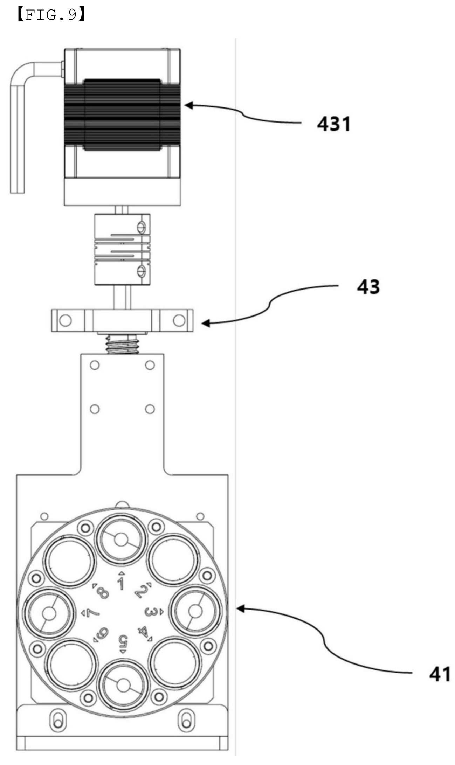

Referring to FIG. 9, the rotary collection tube mounting unit 41 may be moved forward/backward by a collection tube mounting unit opening/closing electric cylinder 43 to open and close the door.

**[Example 1]**

**Resistance range according to the mixing channel size and the number of channel units in the microfluidic device**

Table 1 shows the resistance at each channel inlet (side inlets and a central inlet) according to the mixing channel size and the number of channel units in the microfluidic device of the present invention. Even if the mixing channel size is the same, the resistance may vary depending on the number of units. The dynamic viscosity of all channel sections was set at 20°C.

[TABLE 1]

| Resistance values according to the size and the number of channel units in the microfluidic device | | | | | |
|---|---|---|---|---|---|
| Number of channel units | | X1 (single-unit) | X1 (6-unit) | X3 (single-unit) | X3 (18-unit) |
| Microchannel size | H (mm) | 0.2 | 0.2 | 0.2 | 0.2 |
| | W (mm) | 0.2 | 0.2 | 0.6 | 0.6 |
| | L (mm) | 8 | 10.9 | 6 | 5.7 |
| (Resistance) (Pa·s/m$^3$) | Aqueous phase (Side inlets) | $8.1\times10^{10}$ | $1.8\times10^{10}$ | $9.5\times10^{9}$ | $5.3\times10^{8}$ |
| | Organic phase (central inlet) | $1.6\times10^{11}$ | $3.7\times10^{10}$ | $1.9\times10^{10}$ | $1.1\times10^{9}$ |
| H: Microchannel height W: Microchannel width L: Microchannel length | | | | | |

**[Example 2]**

**Nanoparticle synthesis operating pressure range according to the type of microfluidic device**

The flow rates and pressures required to operate the microfluidic devices of the present invention, based on the Reynolds number, are shown in Tables 2 to 9, according to the type of microfluidic device.

In particular, Tables 3 and 4 show the pressure, flow rate, and Reynolds number applied to each microchannel in a parallel microfluidic device consisting of six units, while Tables 8 and 9 show the pressure, flow rate, and Reynolds number applied to each microchannel in a parallel microfluidic device consisting of 18 units.

[TABLE 2]

| Pressure and Reynolds number according to required flow rates at the side inlets of an X1 (single-unit) microfluidic device | | | |
|---|---|---|---|
| X1 (single-unit) | | | |
| Aqueous phase (side inlets) | | | |
| Pressure (Pa) | Flow rate [all side inlets] | | Re |
| | $10^{-9}$ m$^3$/s | mL/min | |
| 760 | 9.3 | 0.56 | 10 |
| 1,500 | 19 | 1.1 | 20 |
| 3,000 | 37 | 2.2 | 40 |
| 6,000 | 73 | 4.4 | 80 |
| 12,000 | 150 | 8.8 | 160 |
| 23,000 | 280 | 17 | 300 |
| 38, 000 | 470 | 28 | 500 |

[TABLE 3]

| Pressure and Reynolds number according to required flow rate at the central inlet of the X1 (single-unit) microfluidic device | | | |
|---|---|---|---|
| X1 (single-unit) | | | |
| Organic phase (central inlet) | | | |
| Pressure (Pa) | Flow rate | | Re |
| | $10^{-9}$ m$^3$/s | mL/min | |
| 280 | 1.7 | 0.10 | 10 |
| 550 | 3.4 | 0.20 | 20 |
| 1,100 | 6.7 | 0.40 | 40 |
| 2,200 | 13 | 0.80 | 80 |
| 4,300 | 27 | 1.6 | 160 |
| 8,300 | 51 | 3.1 | 300 |
| 14,000 | 85 | 5.1 | 500 |

[TABLE 4]

| Pressure and Reynolds number according to required flow rates at the side inlets of an X1 (six-unit) microfluidic device | | | |
|---|---|---|---|
| X1 (6-unit) | | | |
| Aqueous phase (side inlets) | | | |
| Pressure (Pa) | Flow rate [all side inlets] | | Re |
| | $10^{-9}$ m$^3$/s | mL/min | |
| 1,000 | 56 | 3.4 | 10 |
| 2,000 | 110 | 6.7 | 20 |
| 4,000 | 220 | 13 | 40 |
| 8,100 | 440 | 26 | 80 |
| 16,000 | 880 | 53 | 160 |
| 31,000 | 1,700 | 100 | 300 |
| 52,000 | 2,800 | 170 | 500 |

[TABLE 5]

| Pressure and Reynolds number according to the required flow rate at the central inlet of the X1 (six-unit) microfluidic device | | | |
|---|---|---|---|
| X1 (6-unit) | | | |
| Organic phase (central inlet) | | | |
| Pressure (Pa) | Flow rate | | Re |
| | $10^{-9}$ m$^3$/s | mL/min | |
| 380 | 10 | 0.61 | 10 |
| 750 | 20 | 1.2 | 20 |
| 1,500 | 40 | 2.4 | 40 |
| 3,000 | 80 | 4.8 | 80 |
| 5,900 | 160 | 9.6 | 160 |
| 12,000 | 310 | 18 | 300 |
| 19,000 | 510 | 31 | 500 |

[TABLE 6]

| Pressure and Reynolds number according to the required flow rate at the side inlets of an X3 (single-unit) microfluidic device | | | |
|---|---|---|---|
| X3 (single-unit) | | | |
| Aqueous phase (side inlets) | | | |
| Pressure (Pa) | Flow rate [all side inlets] | | Re |
| | $10^{-9}$ m$^3$/s | mL/min | |
| 2,500 | 260 | 16 | 100 |
| 3,700 | 390 | 24 | 150 |
| 5,000 | 530 | 32 | 200 |
| 7,500 | 790 | 47 | 300 |
| 13,000 | 1,300 | 79 | 500 |

[TABLE 7]

| Pressure and Reynolds number according to the required flow rate at the central inlet of the X3 (single-unit) microfluidic device | | | |
|---|---|---|---|
| X3 (single-unit) | | | |
| Organic phase (central inlet) | | | |
| Pressure (Pa) | Flow rate | | Re |
| | $10^{-9}$ m³/s | mL/min | |
| 900 | 47 | 2.9 | 100 |
| 1,400 | 74 | 4.3 | 150 |
| 1,800 | 95 | 5.7 | 200 |
| 2,800 | 145 | 8.6 | 300 |
| 4,600 | 240 | 14 | 500 |

[TABLE 8]

| Pressure and Reynolds number according to the required flow rate at all 36 side inlets of an X3 (18-unit) microfluidic device | | | |
|---|---|---|---|
| X3 (18-unit) | | | |
| Aqueous phase (side inlets) | | | |
| Pressure (Pa) | Flow rate [all 36 side inlets] | | Re |
| | $10^{-9}$ m³/s | mL/min | |
| 2,500 | 4,700 | 280 | 100 |
| 3,800 | 7,100 | 430 | 150 |
| 5,000 | 9,400 | 570 | 200 |
| 7,600 | 14,000 | 850 | 300 |
| 13,000 | 24,000 | 1,400 | 500 |

[TABLE 9]

| Pressure and Reynolds number according to the required flow rate at all 18 central inlets of the X3 (18-unit) microfluidic device | | | |
|---|---|---|---|
| X3 (18-unit) | | | |
| Organic phase (central inlet) | | | |
| Pressure (Pa) | Flow rate [all 18 central inlets] | | Re |
| | $10^{-9}$ m³/s | mL/min | |
| 900 | 850 | 52 | 100 |
| 1,400 | 1,300 | 77 | 150 |
| 1,800 | 1,700 | 100 | 200 |
| 2,800 | 2,600 | 160 | 300 |
| 4,600 | 4,300 | 260 | 500 |

The pressures shown in Tables 2 to 9 were calculated using the equation below according to the resistance of each microfluidic channel shown in Table 1.

$$Q\left(\frac{m^3}{s}\right) = P\ (Pa) \times \frac{1}{R}\ \left(\frac{m^3}{Pa \cdot s}\right)$$

In the above equation, Q represents the flow rate, P represents the pressure, and R represents the resistance.

Therefore, as shown in Tables 2 to 9, since the resistance varies depending on the size of the mixing channel and the number of channel units in the microfluidic device of the present invention, different pressures are applied according to the type of microfluidic device.

Furthermore, the pressure must be controlled separately for each channel type (the side inlets and the central inlet). For the X1 (single-unit) microfluidic device, the pressure to be controlled at the side inlets is 760 Pa to 38,000 Pa, and the pressure to be controlled at the central inlet is 280 Pa to 14,000 Pa.

For the X1 (six-unit) microfluidic device, the pressure to be controlled at the side inlets is 1,000 Pa to 52,000 Pa, and the pressure to be controlled at the central inlet is 380 Pa to 19,000 Pa.

For the X3 (single-unit) microfluidic device, the pressure to be controlled at the side inlets is 2,500 Pa to 13,000 Pa, and the pressure to be controlled at the central inlet is 900 Pa to 4,600 Pa.

For the X3 (18-unit) microfluidic device, the pressure to be controlled at the side inlets is 2,500 Pa to 13,000 Pa, and the pressure to be controlled at the central inlet is 900 Pa to 4,600 Pa.

**[Example 3]**

**Optimization of the operating pressure range for nanoparticle synthesis according to the type of microfluidic device**

As shown in Tables 2 to 9, the Reynolds number in the X1 microfluidic device range from 10 to 500, and in the X3 microfluidic device range from 100 to 500. However, to ensure uniform nanoparticle synthesis and a mixing efficiency of 90% or more, the Reynolds number in the X1 microfluidic device must be 20 or more, and in the X3 microfluidic device, the Reynolds number must be 150 or more.

In addition, when using biomolecules as raw materials for nanoparticle production, it is preferable to synthesize the nanoparticles within a shear rate of $10^5$ $s^{-1}$ or less to minimize damage to the biomolecule.

Table 10 below shows the shear rate according to each Reynolds number. It can be seen that the shear rate is $10^5$ $s^{-1}$ or less at Reynolds numbers of 300 or less.

[TABLE 10]

| Shear rate according to Reynolds number | |
| --- | --- |
| Re | Shear rate ($10^5$ $s^{-1}$) |
| 10 | 0.03 |
| 20 | 0.07 |
| 40 | 0.1 |
| 80 | 0.3 |
| 160 | 0.5 |
| 300 | 1.0 |
| 500 | 2.0 |

Therefore, when using the X1 (single-unit) microfluidic device, the pressures at which nanoparticles can be synthesized are 1,500 to 38,000 Pa for the side inlets and 550 to 14,000 Pa for the central inlet. When using the X1 (six-unit) microfluidic device, the pressures at which nanoparticles can be synthesized are 2,000 to 52,000 Pa for the side inlets and 750 to 19,000 Pa for the central inlet.

In addition, when using the X1 (single-unit) microfluidic device, the pressures at which nanoparticles can be synthesized while minimizing damage to biomaterials are 1,500 to 23,000 Pa for the side inlets and 550 to 8,300 Pa for the central inlet. When using the X1 (six-unit) microfluidic device, the pressures at which nanoparticles can be synthesized while minimizing damage to biomaterials are 2,000 to 31,000 Pa for the side inlets and 750 to 12,000 Pa for the central inlet.

When using the X3 (single-unit) microfluidic device, the pressures at which nanoparticles can be synthesized are 3,700 to 13,000 Pa for the side inlets and 1,400 to 4,600 Pa for the central inlet. When using the X3 (18-unit) microfluidic device, the pressures at which nanoparticles can be synthesized are 3,800 to 13,000 Pa for the side inlets and 1,400 to 4,600 Pa for the central inlet.

When using the X3 (single-unit) microfluidic device, the pressures at which nanoparticles can be synthesized while minimizing damage to biomaterials are 3,700 to 7,500 Pa for the side inlets and 1,400 to 2,800 Pa for the central inlet. When using the X3 (18-unit) microfluidic device, the pressures at which nanoparticles can be synthesized while minimizing damage to biomaterials are 3,800 to 7,600 Pa for the side inlets and 1,400 to 2,800 Pa for the central inlet.

**[Example 4]**

**Manufacture of nanoparticles depending on lipid mixture concentration using the microfluidic device**

Nanoparticles containing phospholipids (DSPC) and mRNA were manufactured using the microfluidic device of the present invention as follows.

A solution of DSPC (1,2-distearoyl-sn-glycero-3-phosphocholine) in absolute ethanol and an mRNA solution in citrate buffer were prepared. Subsequently, about 1 mL of the DSPC solution in absolute ethanol at a concentration of 0.66 to 20 mg/mL was added to a 50 mL conical tube, and about 3 mL of the mRNA solution in citrate buffer at a concentration of 0.0042 to 0.13 mg/mL was added to another 50 mL conical tube.

For synthesis in the microfluidic device of the present invention, a 50 mL conical tube with the cap fastened was mounted on a precursor rack. Ethanol was flowed through the microfluidic device to clean it, and then ethanol and deionized water were further flowed to minimize air bubbles in the microfluidic device. Thereafter, the injection flow rate of the DSPC solution was set to 0.8 mL/min, and the injection flow rate of the mRNA solution was set to 4.4 mL/min using the microfluidic device of the present invention. Nanoparticles manufactured through the outlet of the microfluidic device of the present invention were obtained, and the obtained nanoparticles were placed in a dialysis cassette and dialyzed for 16 hours at refrigeration temperature or at room temperature.

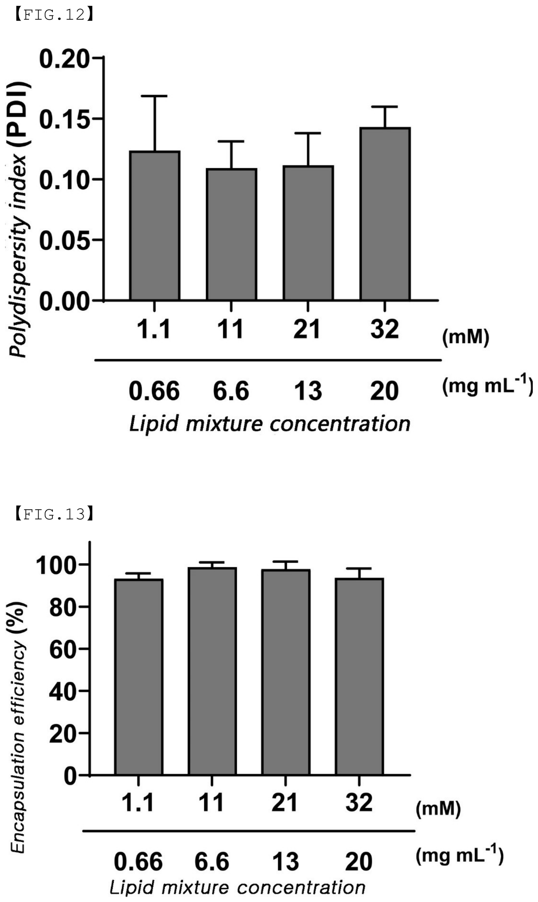

The size distribution of the manufactured nanoparticles was measured using a Zetasizer Nano ZS by dynamic light scattering (DLS), to evaluate changes in particle size due to aggregation of the nanoparticles. The results are shown in Table 11 and FIGS. 11 and 12.

As shown in Table 11 and FIGS. 11 and 12, the sizes of LNPs at lipid mixture concentrations of 0.66, 6.6, 13, and 20 mg/mL were measured to be 55, 70, 72, and 72 nm, respectively. The PDIs at these concentrations were 0.124, 0.109, 0.111, and 0.143, respectively, indicating highly uniform synthesis of the LNPs.

[TABLE 11]

| Lipid mixture concentration (mM) | Lipid mixture concentration (mg/mL) | LNP average size (nm) | PDI | Encapsulation efficiency (%) |
|---|---|---|---|---|
| 1.1 | 0.66 | 55 | 0.124 | 93 |
| 11 | 6.6 | 70 | 0.109 | 99 |
| 21 | 13 | 72 | 0.111 | 98 |
| 32 | 20 | 72 | 0.143 | 94 |

The encapsulation efficiency (EE) was calculated based on the fluorescence intensity of each mRNA LNP, by detecting the total mRNA and the unencapsulated mRNA (free mRNA). The values were calculated according to the following formula:

$$\text{Encapsulation Efficiency (\%)} = \frac{\text{Total mRNA} - \text{Free mRNA}}{\text{Total mRNA}} \times 100.$$

As shown in Table 11 and FIG. 13, the nucleic acid encapsulation efficiencies of the LNPs were 93, 99, 98, and 94%, respectively, at lipid mixture concentrations of 0.66, 6.6, 13, and 20 mg/mL.

It was confirmed that the microfluidic device of the present invention may be used to uniformly synthesize nanoparticles at a desired concentration.

[Description of Reference Numerals]

1: Nanoparticle manufacturing apparatus
10: Cabinet
11: Cabinet upper portion
20: Microfluidic device mounting unit
201: Microfluidic device
21: Mounting unit cover
22: Microfluidic device securing holder
23: Injection valve
30: Inlet unit
31: Raw material tube mounting unit
32: Raw material injection tube
33: Raw material tube sealing electric cylinder
34: Sealing gasket
35: Raw material tube mounting unit opening/closing electric cylinder
351: Electric cylinder motor
36: Pneumatic fitting
40: Collection unit
41: Rotary collection tube mounting unit
42: Rotation motor
43: Collection tube mounting unit opening/closing electric cylinder
431: Electric cylinder motor
50: Pneumatic control unit
51: Primary pneumatic control unit
52: Secondary pneumatic control unit
60: Power supply

**Claims**

1. An apparatus for manufacturing nanoparticles comprising: a microfluidic device; an inlet unit; a collection unit; and a pneumatic control unit.

2. The apparatus for manufacturing nanoparticles according to claim 1, further comprising a microfluidic device mounting unit.

3. The apparatus for manufacturing nanoparticles according to claim 1, further comprising a cabinet.

4. The apparatus for manufacturing nanoparticles according to claim 2, wherein the microfluidic device mounting unit comprises at least one selected from the group consisting of a mounting unit cover, a microfluidic device securing holder, and an injection valve.

5. The apparatus for manufacturing nanoparticles according to claim 1, wherein the inlet unit comprises at least one selected from the group consisting of a tube mounting unit, a raw material injection tube, a sealing electric cylinder, a sealing gasket, a raw material tube mounting unit opening/closing electric cylinder, and a pneumatic fitting.

6. The apparatus for manufacturing nanoparticles according to claim 1, wherein the collection unit comprises at least one selected from the group consisting of a tube mounting unit, a rotation motor, a collection valve, and a collection tube mounting unit opening/closing electric cylinder.

7. The apparatus for manufacturing nanoparticles according to claim 1, wherein the pneumatic control unit comprises at least one selected from the group consisting of a primary pneumatic control unit and a secondary pneumatic control unit.

8. The apparatus for manufacturing nanoparticles according to claim 4, wherein the microfluidic device is secured to a microfluidic device securing holder.

9. The apparatus for manufacturing nanoparticles according to claim 1, wherein the inlet unit and the collection unit have an openable door structure.

10. The apparatus for manufacturing nanoparticles according to claim 7, wherein a gas at a constant pressure is supplied to the inlet unit through the primary pneumatic control unit and the secondary pneumatic control unit.

11. The apparatus for manufacturing nanoparticles according to claim 10, wherein the gas is supplied from outside of the apparatus.

12. The apparatus for manufacturing nanoparticles according to claim 4, wherein the injection valve is configured to control the injection of fluid delivered from the inlet unit.

13. The apparatus for manufacturing nanoparticles according to claim 5, wherein the raw material injection tube is sealed from outside air by the sealing electric cylinder and the sealing gasket.

14. The apparatus for manufacturing nanoparticles according to claim 5, wherein the pressure of a fluid controlled by the pneumatic control unit is transmitted to the microfluidic device through the pneumatic fitting and the raw material injection tube.

15. The apparatus for manufacturing nanoparticles according to claim 5, wherein the raw material tube mounting unit is moved forward and backward by the raw material tube mounting unit opening/closing electric cylinder to open and close a door.

16. The apparatus for manufacturing nanoparticles according to claim 6, wherein the collection tube mounting unit is rotated by the rotation motor, and nanoparticles synthesized in the microfluidic device may be stored in a separate collection tube.

17. The apparatus for manufacturing nanoparticles according to claim 6, wherein the collection tube mounting unit is moved forward and backward by the collection tube mounting unit opening/closing electric cylinder to open and close a door.

18. The apparatus for manufacturing nanoparticles according to claim 1, wherein the microfluidic device comprises one or more microchannels.

19. The apparatus for manufacturing nanoparticles according to claim 18, wherein the height-to-width ratio of the microchannel is 1:1 to 1:5.

20. The apparatus for manufacturing nanoparticles according to claim 18, wherein the Reynolds number in the microchannel is 10 to 500.

21. The apparatus for manufacturing nanoparticles according to claim 18, wherein the Reynolds number in the microchannel is 20 to 300.

22. The apparatus for manufacturing nanoparticles according to claim 1, wherein the nanoparticles manufactured by the apparatus for manufacturing nanoparticles have a size of 10 to 100 nm.

23. The apparatus for manufacturing nanoparticles according to claim 1, wherein the polydispersity index of the nanoparticles manufactured by the apparatus for manufacturing nanoparticles is 0.05 to 0.15.

24. The apparatus for manufacturing nanoparticles according to claim 1, wherein the nanoparticles manufactured by the apparatus for manufacturing nanoparticles have an encapsulation efficiency of 90% or more.

25. A method for manufacturing nanoparticles using the apparatus for manufacturing nanoparticles according to claim 1, the method comprising:

    introducing a fluid into the microfluidic device from the inlet unit at a constant flow rate; and
    collecting products produced in the microfluidic device in the collection unit.

26. The method for manufacturing nanoparticles according to claim 25, wherein, as the fluid is introduced into the microfluidic device from the inlet unit, the flow rate of the fluid is controlled by the pneumatic control unit.

27. The method for manufacturing nanoparticles according to claim 25, wherein the products are separated and collected in the collection unit.

【FIG.1a】

【FIG.1b】

【FIG.2】

【FIG.3】

【FIG.4a】

【FIG.4b】

【FIG.5】

【FIG.6a】

【FIG.6b】

【FIG.7a】

【FIG.7b】

【FIG.8a】

【FIG.8b】

【FIG.9】

【FIG.10】

【FIG.11】

【FIG.12】

【FIG.13】

【FIG.14】

【FIG.15】

【FIG.16】

## INTERNATIONAL SEARCH REPORT

| International application No. |
|---|
| **PCT/KR2025/006132** |

| A. | CLASSIFICATION OF SUBJECT MATTER |
|---|---|

**B01J 19/00**(2006.01)i; **A61K 9/51**(2006.01)i; **B01L 9/00**(2006.01)i

According to International Patent Classification (IPC) or to both national classification and IPC

| B. | FIELDS SEARCHED |
|---|---|

Minimum documentation searched (classification system followed by classification symbols)

B01J 19/00(2006.01); B01L 3/00(2006.01); C12M 1/00(2006.01); C12M 1/36(2006.01); G01N 15/00(2006.01); H02P 6/182(2016.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미세유체(microfluid), 나노지질입자(lipid nano particles, LNP), 공압 (pneumatic), 채널(channel)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2023-0160229 A (GPB SCIENTIFIC, INC.) 23 November 2023 (2023-11-23)<br>paragraphs [0049]-[0104]; figures 1-4c | 1-27 |
| Y | KR 10-2023-0009048 A (RADIANQBIO CO., LTD.) 17 January 2023 (2023-01-17)<br>claims 1-7; paragraphs [0011]-[0069]; figures 1-6 | 1-27 |
| Y | CN 112368562 A (THE UNIVERSITY OF HONG KONG) 12 February 2021 (2021-02-12)<br>claims 1-5; paragraph [0021]; figures 1-6 | 6,16,17,27 |
| Y | KR 10-2018-0031034 A (CEPHEID) 27 March 2018 (2018-03-27)<br>paragraphs [0086]-[0091]; figures 4a-4e | 9,15,17 |
| DY | KR 10-2631907 B1 (MEPSGEN CO., LTD.) 31 January 2024 (2024-01-31)<br>claim 1; paragraphs [0018]-[0073], [0154] | 18-23 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 August 2025** | **06 August 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2025/006132**

| Patent document cited in search report | | | Publication date (day/month/year) | Patent family member(s) | | | Publication date (day/month/year) |
|---|---|---|---|---|---|---|---|
| KR | 10-2023-0160229 | A | 23 November 2023 | AU | 2022-209752 | A1 | 03 August 2023 |
| | | | | AU | 2022-209752 | A9 | 17 October 2024 |
| | | | | CA | 3204796 | A1 | 28 July 2022 |
| | | | | CN | 117083371 | A | 17 November 2023 |
| | | | | EP | 4281537 | A1 | 29 November 2023 |
| | | | | EP | 4281537 | A4 | 30 April 2025 |
| | | | | JP | 2024-506485 | A | 14 February 2024 |
| | | | | US | 2024-0101944 | A1 | 28 March 2024 |
| | | | | WO | 2022-159552 | A1 | 28 July 2022 |
| KR | 10-2023-0009048 | A | 17 January 2023 | None | | | |
| CN | 112368562 | A | 12 February 2021 | EP | 3797279 | A1 | 31 March 2021 |
| | | | | EP | 3797279 | A4 | 03 August 2022 |
| | | | | US | 12239981 | B2 | 04 March 2025 |
| | | | | US | 2021-0260587 | A1 | 26 August 2021 |
| | | | | US | 2025-0144624 | A1 | 08 May 2025 |
| | | | | WO | 2020-011193 | A1 | 16 January 2020 |
| KR | 10-2018-0031034 | A | 27 March 2018 | AU | 2016-297866 | A1 | 08 March 2018 |
| | | | | AU | 2016-297866 | B2 | 21 October 2021 |
| | | | | AU | 2022-200255 | A1 | 10 February 2022 |
| | | | | AU | 2022-200255 | B2 | 29 February 2024 |
| | | | | AU | 2024-201113 | A1 | 14 March 2024 |
| | | | | BR | 112018001352 | A2 | 11 September 2018 |
| | | | | BR | 112018001352 | B1 | 06 October 2020 |
| | | | | CA | 2992831 | A1 | 02 February 2017 |
| | | | | CN | 107923922 | A | 17 April 2018 |
| | | | | CN | 107923922 | B | 22 February 2022 |
| | | | | CN | 114740213 | A | 12 July 2022 |
| | | | | EA | 039230 | B1 | 21 December 2021 |
| | | | | EA | 201890370 | A1 | 31 August 2018 |
| | | | | EP | 3325975 | A1 | 30 May 2018 |
| | | | | HK | 1252768 | A1 | 31 May 2019 |
| | | | | JP | 2018-527019 | A | 20 September 2018 |
| | | | | JP | 2022-109951 | A | 28 July 2022 |
| | | | | JP | 7062587 | B2 | 06 May 2022 |
| | | | | JP | 7589875 | B2 | 26 November 2024 |
| | | | | KR | 10-2650704 | B1 | 22 March 2024 |
| | | | | MX | 2018000788 | A | 05 September 2018 |
| | | | | MX | 2021008845 | A | 08 September 2021 |
| | | | | MX | 389991 | B | 19 March 2025 |
| | | | | US | 10562030 | B2 | 18 February 2020 |
| | | | | US | 11524301 | B2 | 13 December 2022 |
| | | | | US | 12280379 | B2 | 22 April 2025 |
| | | | | US | 2017-0021356 | A1 | 26 January 2017 |
| | | | | US | 2020-0188922 | A1 | 18 June 2020 |
| | | | | US | 2023-0166263 | A1 | 01 June 2023 |
| | | | | WO | 2017-019569 | A1 | 02 February 2017 |
| | | | | ZA | 201801050 | B | 26 June 2024 |
| KR | 10-2631907 | B1 | 31 January 2024 | WO | 2024-205076 | A1 | 03 October 2024 |

Form PCT/ISA/210 (patent family annex) (July 2022)

## REFERENCES CITED IN THE DESCRIPTION

**Patent documents cited in the description**

- KR 102492420

- KR 102631907

**Non-patent literature cited in the description**

- **VALENCIA, P. et al.** Single-Step Assembly of Homogenous Lipid-Polymeric and Lipid-Quantum Dot Nanoparticles Enabled by Microfluidic Rapid Mixing. *ACS Nano*, 2010, vol. 4 (3), 1671-1679

- **RHEE, M. et al.** Drop Mixing in a Microchannel for Lab-on-a-Chip Platforms.. *Langmuir*, 2008, vol. 24 (2), 590-601
- **ROHIT, K et al.** Microfluidic Platform for Controlled Synthesis of Polymeric Nanoparticles.. *Nano Letters*, 2008, vol. 8 (9), 2906-12